# EUROPEAN PATENT SPECIFICATION

(11) **EP 1 750 780 B1**
(45) Date of publication and mention of the grant of the patent: **03.10.2007**
(21) Application number: 05851181.7
(22) Date of filing: 12.05.2005
(51) Int. Cl.: A61L 27/36, A61L 27/54, A61L 29/08, A61L 29/14, A61L 31/14, A61L 31/10

(54) **MEDICAL DEVICES AND METHODS OF MAKING THE SAME**
MEDIZINISCHE VORRICHTUNGEN UND VERFAHREN ZU IHRER HERSTELLUNG
DISPOSITIFS MEDICAUX ET PROCEDES DE FABRICATION DES DISPOSITIFS

(30) Priority: 20.05.2004 US 849742; 10.11.2004 US 985242
(43) Date of publication of application: 14.02.2007
(73) Proprietor: Boston Scientific Limited, Barbados, West Indies (BB)
(72) Inventor: WEBER, Jan, Maple Grove, Minnesota 55311 (US)
(74) Representative: Brady, Paul Andrew
(86) International application number: PCT/US2005/016600
(87) International publication number: WO 2006/060033

(56) References cited:
- EP-A- 0 972 563
- WO-A-01/49338
- WO-A-01/78906
- WO-A-03/035278

## Description

### TECHNICAL FIELD

The invention relates to medical devices, such as, for example, endoprostheses, and methods of making the devices.

### BACKGROUND

The body includes various passageways such as arteries, other blood vessels, and other body lumens. These passageways sometimes become occluded or weakened. For example, the passageways can be occluded by a tumor, restricted by plaque, or weakened by an aneurysm. When this occurs, the passageway can be reopened or reinforced, or even replaced, with a medical endoprosthesis. An endoprosthesis is typically a tubular member that is placed in a lumen in the body. Examples of endoprostheses include stents, stent-grafts, and covered stents.

An endoprosthesis can be delivered inside the body by a catheter that supports the endoprosthesis in a compacted or reduced-size form as the endoprosthesis is transported to a desired site. Upon reaching the site, the endoprosthesis is expanded, for example, so that it can contact the walls of the lumen.

The expansion mechanism may include forcing the endoprosthesis to expand radially. For example, the expansion mechanism can include the catheter carrying a balloon, which carries a balloon-expandable endoprosthesis. The balloon can be inflated to deform and to fix the expanded endoprosthesis at a predetermined position in contact with the lumen wall. The balloon can then be deflated, and the catheter withdrawn.

In another delivery technique, the endoprosthesis is formed of an elastic material that can be reversibly compacted and expanded (e.g., elastically or through a material phase transition). During introduction into the body, the endoprosthesis is restrained in a compacted condition. Upon reaching the desired implantation site, the restraint is removed, for example, by retracting a restraining device such as an outer sheath, enabling the endoprosthesis to self-expand by its own internal elastic restoring force.

To support a passageway and keep the passageway open, endoprostheses are sometimes made of relatively strong materials, such as stainless steel or Nitinol (a nickel-titanium alloy), formed into struts or wires.

### SUMMARY

In one aspect, the invention features medical devices (e.g., endoprostheses) that include one or more biodisintegrable materials, and methods of making the devices. The medical devices may include at least one region that is formed of one or more biodisintegrable materials, or may be formed entirely of one or more biodisintegrable materials. The disintegration of the biodisintegrable materials in the medical devices can be controlled. For example, in some embodiments, the medical devices can include one or more polyelectrolytes, which can be used to control the disintegration of the biodisintegrable materials. In certain embodiments, the polyelectrolytes can be in the form of layers. The layers can, for example, limit or prevent water molecules and/or certain ions (e.g., in body fluid) from contacting the biodisintegrable materials, thereby delaying and/or slowing the disintegration of the biodisintegrable materials. In some embodiments, the layers can limit the movement of the water molecules and/or ions, and/or can slow the rate at which the water molecules and/or ions access and contact the biodisintegrable materials.

In another aspect, the invention features a medical device including a tubular body that includes a biodisintegrable material, and a first polyelectrolyte on the tubular body.

In an additional aspect, the invention features a medical device that includes a substrate including a biodisintegrable material and a layer supported by the substrate, the layer including one or more charges.

A method of making the above medical device, includes forming a first layer on a tubular body including a biodisintegrable material. The first layer includes a polyelectrolyte.

In an additional aspect, the invention features a medical device including a tubular body that includes a biodisintegrable material, a polyelectrolyte on the tubular body, and a non-polyelectrolyte biodisintegrable coating.

Embodiments may include one or more of the following features.

The polyelectrolyte can be biodisintegrable.

In some embodiments, the polyelectrolyte can be a polyacid, such as a polyphosphoric acid, a polyvinylsulfuric acid, a polyvinylsulfonic acid, a polyvinylphosphonic acid, or a polyacrylic acid. In certain embodiments, the polyelectrolyte can be a polybase, such as polyallylamine, polyethylimine, polyvinylamine, or polyvinylpyridine.

In some embodiments, the polyelectrolyte can be a polycation, such as a poly(allylamine) polycation, a polyethyleneimine polycation, a polydiallyldimethylammonium polycation, a protamine sulfate polycation, a chitosan polycation, a gelatin polycation, a spermidine polycation, a poly(*N*-octyl-4-vinyl pyridinium iodide) polycation, or an albumin polycation. In certain embodiments, the polyelectrolyte can be a polyanion, such as a poly(styrene sulfonate) polyanion, a polyacrylic acid polyanion, a sodium alginate polyanion, a polystyrene sulfonate polyanion, a eudragit polyanion, a gelatin polyanion, a hyaluronic acid polyanion, a carrageenan polyanion, a chondroitin sulfate polyanion, or a carboxymethylcellulose polyanion.

The polyelectrolyte can include a polymer, such as heparin, a protein, polyglycolic acid, polylactic acid, a polyamide, poly-2-hydroxy-butyrate, polycaprolactone, or poly(lactic-co-glycolic)acid.

The layer can include a polyelectrolyte. The layer can be biodisintegrable or biostable.

The medical device can have a first layer that includes the polyelectrolyte. The first layer can be supported by the generally tubular body. In some embodiments, the first layer can contact the tubular body. The first layer can have a thickness of at most 500 nanometers (e.g., at most 300 nanometers, at most 100 nanometers, at most 50 nanometers, at most 10 nanometers, at most five nanometers, at most one nanometer, at most 0.5 nanometer) and/or at least 0.2 nanometer (e.g., at least 0.5 nanometer, at least one nanometer, at least five nanometers, at least 10 nanometers, at least 50 nanometers, at least 100 nanometers, at least 300 nanometers). In some embodiments, the first layer can have at least one (e.g., at least two, at least five, at least 10) opening in it (e.g., in a wall of the first layer).

The medical device can further include a second layer that can be supported by the tubular body. In some embodiments, the second layer can contact the first layer. The second layer can include a polyelectrolyte that can be the same as, or different from, the polyelectrolyte in the first layer. In certain embodiments, the polyelectrolyte in one layer of the medical device can be positively charged and the polyelectrolyte in another layer (e.g., an adjacent layer) of the medical device can be negatively charged.

In some embodiments, the medical device can include a plurality of layers, such as a plurality of first layers and a plurality of second layers. In certain embodiments, at least two of the first layers can be spaced from each other by one or more second layers.

In certain embodiments, the medical device can further include a second polyelectrolyte on the generally tubular body. In some embodiments, the first polyelectrolyte and the second polyelectrolyte can be cross-linked to each other (e.g., by at least one covalent bond).

In certain embodiments, the non-polyelectrolyte biodisintegrable coating may cover all or a portion of the polyelectrolyte.

In some embodiments, the method can include forming a second layer on the first layer. The second layer can include a polyelectrolyte. In certain embodiments, the method can include cross-linking the polyelectrolyte in the first layer to the polyelectrolyte in the second layer (e.g., by applying heat and/or UV radiation to the first and second layers). In certain embodiments, the method can include forming a plurality of first layers and a plurality of second layers.

The medical device can include at least 10 layers (e.g., at least 20 layers, at least 50 layers, at least 100 layers, at least 200 layers), and/or at most 300 layers (e.g., at most 200 layers, at most 100 layers, at most 50 layers, at most 20 layers). The plurality of layers can have a total thickness of at most 1500 nanometers (e.g., at most 1000 nanometers, at most 500 nanometers, at most 100 nanometers, at most 50 nanometers, at most 10 nanometers), and/or at least five nanometers (e.g., at least 10 nanometers, at least 50 nanometers, at least 100 nanometers, at least 500 nanometers, at least 1000 nanometers). In some embodiments, one portion of the medical device can include a greater number of layers than another portion of the medical device. In certain embodiments, one portion of the medical device can include at least 10 layers (e.g., at least 20 layers, at least 30 layers, at least 40 layers), and/or another portion of the medical device can include at least 20 layers (e.g., at least 30 layers, at least 40 layers, at least 50 layers). In some embodiments, one portion of the medical device can include 10 layers while another portion of the medical device can include 20 layers.

The medical device can be an endoprosthesis, a catheter, a graft, or a filter.

The biodisintegrable material can be a metal, a metal alloy, and/or a non-metal. In some embodiments, the biodisintegrable material can be an alkali metal, an alkaline earth metal (e.g., magnesium), iron, zinc, aluminum, or an alloy (e.g., an iron alloy, a magnesium alloy). In certain embodiments, the biodisintegrable material can include one component (e.g., magnesium, titanium, zirconium, niobium, tantalum, zinc, silicon), and another, different, component (e.g., lithium, sodium, potassium, calcium, iron, manganese). In some embodiments, the biodisintegrable material can be a polymer.

Embodiments may include one or more of the following advantages.

In certain embodiments, the medical device may be used to temporarily treat a subject without permanently remaining in the body of the subject. For example, in some embodiments, the medical device can be used for a certain period of time (e.g., to support a lumen of a subject), and then can disintegrate after that period of time. In certain embodiments, as a medical device disintegrates, the Magnetic Resonance Imaging (MRI) visibility and/or Computed Tomography (CT) visibility of the region (e.g., a body lumen) in which the medical device is located can increase.

In some embodiments in which the medical device includes one or more polyelectrolytes, the polyelectrolytes can be used to control the disintegration (e.g., corrosion) of biodisintegrable materials in the medical device. The polyelectrolytes can, for example, be in the form of layers that can limit or prevent water molecules and/or certain ions from accessing the biodisintegrable materials. In certain embodiments, the polyelectrolytes can prevent the biodisintegrable materials from disintegrating prematurely (e.g., during delivery and/or deployment of the medical device to a target site).

In certain embodiments in which the medical device includes one or more polyelectrolyte layers, different regions of the medical device can include different numbers of polyelectrolyte layers. In some embodiments, the biodisintegrable material in a region of the medical device that includes a relatively high number of polyelectrolyte layers may begin to disintegrate after, and/or more slowly than, the biodisintegrable material in a region of the medical device that includes a relatively low number of polyelectrolyte layers. Thus, the polyelectrolyte layers on a medical device may be used to provide different disintegration rates of biodisintegrable material in different regions of the medical device. In some embodiments, an endoprosthesis can include an arrangement of polyelectrolyte layers that causes one or both of the ends of the endoprosthesis to start disintegrating before the middle of the endoprosthesis. This may limit the likelihood of the medical device breaking apart into two or more pieces during disintegration.

In certain embodiments, an endoprosthesis can include two or more polyelectrolyte layers that are cross-linked to each other. The cross-linked polyelectrolyte layers may be used, for example, to confine biodisintegrable material in the endoprosthesis. In certain embodiments, this confinement of the biodisintegrable material may limit the likelihood that one or more pieces of the biodisintegrable material will break away from the endoprosthesis during use and move to a location other than the target site.

Other aspects, features, and advantages are in the description, drawings, and claims.

### DESCRIPTION OF DRAWINGS

FIG 1 is a perspective view of an embodiment of an endoprosthesis.
FIG 2 is a detailed cross-sectional illustration of the endoprosthesis of FIG 1, taken along line 2-2.
FIG. 3 is a flow chart of an embodiment of a method of making a medical device.
FIG. 4 is a perspective view of an embodiment of an endoprosthesis.
FIG. 5 is a detailed cross-sectional illustration of the endoprosthesis of FIG. 4, taken along line 5-5.
FIG. 6 is a detailed cross-sectional illustration of the endoprosthesis of FIG. 4, taken along line 6-6.

### DETAILED DESCRIPTION

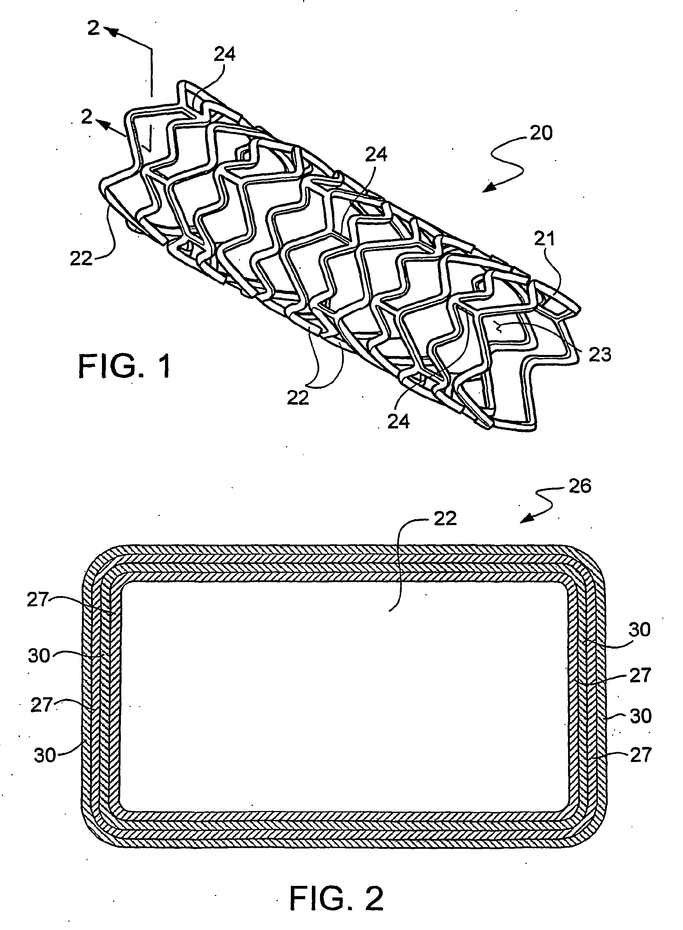

FIG. 1 shows a stent 20 that is in the form of a tubular structure 21 including a biodisintegrable material. Tubular structure 21 is defined by a plurality of bands 22 and a plurality of connectors 24 that extend between and connect adjacent bands. Tubular structure 21 has a lumen 23. As shown in FIG. 2, stent 20 (as shown, a portion of a band 22) is coated with a multi-layered structure 26 that includes a plurality of first charged layers 27 and a plurality of second charged layers 30. Multi-layered structure 26 is capable of delaying and/or slowing the disintegration of tubular structure 21 (e.g., during delivery and/or use of stent 20 at a target site).

In some embodiments, layers 27 and/or 30 can include one or more polyelectrolytes. For example, layers 27 can include a charged polyelectrolyte and layers 30 can include an oppositely charged polyelectrolyte, to maintain charge balance with the charged polyelectrolyte in layers 27. Each of layers 27 and/or layers 30 may include one type of polyelectrolyte or may include multiple different types of polyelectrolytes. It is believed that polyelectrolyte anion/cation pairs in layers 27 and 30 can bind water molecules and certain ions (e.g., chlorine ions, metal ions), thereby limiting or preventing movement of the water molecules and ions, and/or limiting or preventing the water molecules and ions from contacting tubular structure 21 of stent 20.

The movement of water molecules and ions within layers 27 and 30, and/or the extent of contact between tubular structure 21 of stent 20 and the water molecules and ions, may depend on any of a number of different factors.

As an example, in certain embodiments, as the electrostatic attraction between the anion/cation pairs in layers 27 and 30 increases, the strength of the bonds holding the water molecules and ions can also increase. As a result, the movement of water molecules and ions within layers 27 and 30 may decrease, and/or tubular structure 21 of stent 20 may have less contact with water molecules and ions. The relative electrostatic attraction between anion/cation pairs in polyelectrolyte layers can be estimated by, for example, measuring the zeta potential of one polyelectrolyte layer and then measuring the zeta potential of another polyelectrolyte layer that is formed upon the first polyelectrolyte layer. In some embodiments, the electrostatic attraction between anion/cation pairs in adjacent polyelectrolyte layers can be relatively high, such as embodiments in which the anionic polyelectrolyte layer has a zeta potential of less than -40 millivolts and the cationic polyelectrolyte layer has a zeta potential of more than 40 millivolts. The zeta potential of a polyelectrolyte can be measured using laser doppler electrophoresis (e.g., using a ZetaPALS system from Brookhaven Instruments Corp., Holtsville, NY).

As another example, in some embodiments, as the temperature of the environment surrounding stent 20 increases, the movement of water molecules and ions within layers 27 and 30 may increase, and/or tubular structure 21 of stent 20 may have less contact with water molecules and ions. In some embodiments in which layers 27 and/or 30 are biodisintegrable, as described below, the increased temperature may result in a higher disintegration rate of layers 27 and/or 30, allowing increased access of water molecules and ions to tubular structure 21 of stent 20.

As an additional example, in certain embodiments, as the pH of the environment surrounding stent 20 increases, the movement of water molecules and ions within layers 27 and 30 may decrease, and/or the amount of contact between water molecules and ions and tubular structure 21 of stent 20 may decrease. The pH of the environment surrounding stent 20 may change, for example, as the biodisintegrable material in tubular structure 21 disintegrates. For example, in some embodiments in which stent 20 includes magnesium, oxidation of the magnesium can cause the pH of the environment surrounding stent 20 to increase.

Polyelectrolytes are polymers having charged (e.g., ionically dissociable) groups. The number of these groups in the polyelectrolytes can be so large that the polymers are soluble in polar solvents (including water) when in ionically dissociated form (also called polyions). Depending on the type of dissociable groups, polyelectrolytes can be classified as polyacids or polybases.

When dissociated, polyacids form polyelectrolyte anions (polyanions), with protons being split off. Polyacids include inorganic, organic and biopolymers. Examples of polyacids include polyphosphoric acids, polyvinylsulfuric acids, polyvinylsulfonic acids, polyvinylphosphonic acids and polyacrylic acids. Examples of the corresponding salts, which are called polysalts, include polyphosphates, polyvinylsulfates, polyvinylsulfonates, polyvinylphosphonates and polyacrylates.

Polybases contain groups that are capable of accepting protons (e.g., by reaction with acids), with a salt being formed. Examples of polybases having dissociable groups within their backbone and/or side groups include polyallylamine, polyethyleneimine, polyvinylamine and polyvinylpyridine. By accepting protons, polybases form polyelectrolyte cations (polycations).

Some polyelectrolytes have both anionic and cationic groups, but nonetheless have a net positive or negative charge. An example of such a polyelectrolyte is gelatin. Whether a polyelectrolyte having both anionic and cationic groups has a net positive or negative charge can depend, for example, of the pH of the environment surrounding the polyelectrolyte.

In some embodiments, polyelectrolytes can be based on biopolymers. Examples include alginic acid, gummi arabicurn, nucleic acids, pectins and proteins, chemically modified biopolymers such as carboxymethylcellulose and lignin sulfonates, and synthetic polymers such as polymethacrylic acid, polyvinylsulfonic acid, polyvinylphosphonic acid and polyethyleneimine.

Polyelectrolytes may be linear or branched. The use of branched polyelectrolytes on stent 20 can lead to less compact polyelectrolyte multilayers having a higher degree of wall porosity. In some embodiments, polyelectrolyte molecules can be crosslinked within and/or between individual polyelectrolyte layers (e.g., by crosslinking amino groups with aldehydes). The cross-linking may enhance the stability of the polyelectrolyte layers.

In certain embodiments, polyelectrolytes can be amphiphilic. For example, polyelectrolytes may include amphiphilic block or random copolymers having partial polyelectrolyte character. Amphiphilic polyelectrolytes can be used, for example, to affect permeability towards polar molecules. In some embodiments, a layer including an amphiphilic polyelectrolyte may be more permeable to polar molecules than a layer including a polyelectrolyte that is not amphiphilic.

Other examples of polyelectrolytes include low-molecular weight polyelectrolytes (e.g., polyelectrolytes having molecular weights of a few hundred Daltons) up to macromolecular polyelectrolytes (e.g., polyelectrolytes of synthetic or biological origin, which commonly have molecular weights of several million Daltons).

Specific examples of polyelectrolyte cations (polycations) include protamine sulfate polycations, poly(allylamine) polycations (e.g., poly(allylamine hydrochloride) (PAH)), polydiallyldimethylammonium polycations, polyethyleneimine polycations, chitosan polycations, gelatin polycations, spermidine polycations and albumin polycations.

Specific examples of polyelectrolyte anions (polyanions) include poly(styrenesulfonate) polyanions (e.g., poly(sodium styrene sulfonate) (PSS)), polyacrylic acid polyanions, sodium alginate polyanions, eudragit polyanions, gelatin polyanions, hyaluronic acid polyanions, carrageenan polyanions, chondroitin sulfate polyanions, and carboxymethylcellulose polyanions.

In some embodiments, biodisintegrable polyelectrolytes can be used in layers 27 and/or 30. As used herein, a "biodisintegrable material" is a material that undergoes dissolution, degradation, absorption, erosion, corrosion, resorption and/or other disintegration processes over the period that a device including the biodisintegrable material is designed to reside in a patient. As the biodisintegrable polyelectrolytes in layers 27 and/or 30 disintegrate, they may provide less protection for tubular structure 21 of stent 20. As a result, tubular structure 21 can begin to disintegrate or can disintegrate at a faster rate.

Examples of biodisintegrable polyelectrolytes include heparin, polyglycolic acid (PGA), polylactic acid (PLA), polyamides, poly-2-hydroxy-butyrate (PHB), polycaprolactone (PCL), poly(lactic-co-glycolic)acid (PLGA), protamine sulfate, polyallylamine, polydiallyldimethylammonium species (e.g., poly(diallyldimethylammonium chloride) (PDADMA, Aldrich)), polyethyleneimine, chitosan, eudragit, gelatin, spermidine, albumin, polyacrylic acid, sodium alginate, poly(styrene sulfonate) (PSS, Scientific Polymer Products), hyaluronic acid, carrageenan, chondroitin sulfate, carboxymethylcellulose, polypeptides, proteins, DNA, and poly(*N*-octyl-4-vinyl pyridinium iodide) (PNOVP). Polyelectrolytes are described, for example, in Tarek R. Farhat and Joseph B. Schlenoff, "Corrosion Control Using Polyelectrolyte Multilayers", Electrochemical and Solid State Letters, 5 (4) B13-B15 (2002).

In some embodiments, a layer formed of a biodisintegrable polyelectrolyte can disintegrate over a period of at least one second (e.g., at least 10 seconds, at least 30 seconds, at least one minute, at least 10 minutes, at least one hour, at least five hours, at least 10 hours, at least one day, at least two days, at least four days, at least six days), and/or most one week (e.g., at most six days, at most four days, at most two days, at most one day, at most 10 hours, at most five hours, at most one hour, at most 10 minutes, at most one minute, at most 30 seconds, at most 10 seconds).

In certain embodiments, a biodisintegrable polyelectrolyte in a layer can be cross-linked (e.g., using heat and/or UV radiation) to another biodisintegrable polyelectrolyte in another layer. In some embodiments, cross-linking between polyelectrolytes in different layers can cause the polyelectrolytes to disintegrate at a slower rate than they would otherwise. In certain embodiments, a layer including a cross-linked polyelectrolyte can disintegrate over a period of at least one week (e.g., at least two weeks, at least three weeks, at least four weeks, at least six weeks, at least eight weeks, at least 10 weeks, at least 12 weeks, at least 14 weeks, at least 16 weeks, at least 18 weeks, at least 20 weeks, at least 22 weeks), and/or at most 24 weeks (e.g., at most 22 weeks, at most 20 weeks, at most 18 weeks, at most 16 weeks, at most 14 weeks, at most 12 weeks, at most 10 weeks, at most eight weeks, at most six weeks, at most four weeks, at most three weeks, at most two weeks).

As the biodisintegrable polyelectrolytes disintegrate, the biodisintegrable material of tubular structure 21 can, for example, become more exposed to water. This increased exposure to water can cause tubular structure 21 to begin to disintegrate or to disintegrate more rapidly. Eventually, tubular structure 21 may disintegrate entirely.

In certain embodiments, biodisintegrable polyelectrolytes can be used near the outer surface of stent 20 to release a therapeutic agent into the subject at a rate that is dependent upon the rate of disintegration of the polyelectrolyte layers. Examples of therapeutic agents are described below.

In some embodiments, layers 27 and/or 30 may include biodisintegrable polyelectrolytes that may disintegrate at a slower rate than the biodisintegrable material of tubular structure 21 of stent 20. As tubular structure 21 disintegrates, it may break into multiple pieces. Because layers 27 and/or 30 are formed of biodisintegrable materials that disintegrate at a slower rate than the biodisintegrable material of tubular structure 21, at least some of layers 27 and/or 30 may limit or prevent movement of these pieces to other places in the body, causing the pieces to disintegrate instead at the target site within the body.

While layers formed of biodisintegrable polyelectrolytes have been described, in some embodiments, polyelectrolytes in different layers of a medical device can be sufficiently cross-linked to each other to make the layers biostable. As used herein, a "biostable material" is a material that does not undergo substantial dissolution, degradation, corrosion, resorption and/or other disintegration processes over the period that a device including the biostable material is designed to reside in a patient. Thus if, for example, layers 27 and 30 are cross-linked to each other so that multi-layered structure 26 is biostable, tubular structure 21 may disintegrate over a period of time, while multi-layered structure 26 remains in the body of the subject.

As an example, a polyelectrolyte layer including diazonium cations may be covalently cross-linked to a polyelectrolyte layer including sulfonate groups or acrylic acid groups, using UV radiation or heat. As another example, a polyelectrolyte layer including a diazo resin may be cross-linked to a polyelectrolyte layer including polyoxometalates. As an additional example, ammonium groups in one polyelectrolyte layer may be covalently bonded to carboxylate groups in another polyelectrolyte layer. In certain embodiments, polyelectrolyte layers including poly(allylamine hydrochloride) (PAH) can be covalently bonded to polyelectrolyte layers including poly(acrylic acid) (PAA). Cross-linking of polyelectrolyte layers is described, for example, in Zhang et al., "Improving multilayer films endurance by photoinduced interaction between Dawson-type polyoxometalate and diazo resin", Materials Chemistry and Physics 90 (2005), 47-52, and in Zhang et al., "Ways for fabricating stable layer-by-layer self-assemblies: combined ionic self-assembly and post chemical reaction", Colloids and Surfaces A: Physicochemical and Engineering Aspects 198-200 (2002), 439-442.

In some embodiments, one or more of the top polyelectrolyte layers on a medical device can be cross-linked. This can, for example, limit or prevent dissolution of the polyelectrolyte layers on the medical device in the body. For example, multiple layers of polyallylamine hydrochloride (PAH) and polyacrylic acid (PAA) can be deposited on a plurality of other polyelectrolyte layers. The entire multi-layered structure can then be heated at 130°C for about an hour under a nitrogen atmosphere to crosslink the ammonium groups of the PAH and the carboxylic groups of the PAA to form amide bonds. A nylon-like top film that is impermeable to liquids can be created. In certain embodiments, this liquid-impermeable top film can later be removed (e.g., using excimer ablation).

In some embodiments, a liquid-impermeable layer (such as a top layer) may be formed on a biodisintegrable medical device, such as a biodisintegrable stent (e.g., a biodisintegrable abdominal aortic aneurysm (AAA) stent) or a biodisintegrable vessel graft. As an example, in certain embodiments, a biodisintegrable stent (e.g., a biodisintegrable stent formed of iron or magnesium) may have certain desirable properties. For example, magnesium has a relatively low mass attenuation factor, and the CT visibility of the region (e.g., a body lumen) in which a magnesium stent is located can be relatively high. Thus, it may be desirable for the stent not to disintegrate in the body. A liquid-impermeable layer can be formed over the stent to limit or prevent disintegration of the stent.

In certain embodiments in which multi-layered structure 26 is biostable, layers 27 and 30 may have one or more (e.g., two, three, four, five, 10,15,20,25) holes in them. In some embodiments, one or more holes can be added to a layer using a laser. The holes can, for example, provide water and/or ions with limited access to tubular structure 21, thereby helping cause tubular structure 21 to disintegrate. As discussed above, as tubular structure 21 disintegrates, it may break into multiple pieces. Because multi-layered structure 26 is biostable, multi-layered structure 26 may limit or prevent movement of these pieces to other places in the body, causing the pieces to disintegrate instead at the target site within the body. In certain embodiments, a biostable multi-layered structure including one or more holes in at least one of its layers can be coated with a biodisintegrable coating. The biodisintegrable coating can, for example, be used to limit or prevent corrosion of a structure (e.g., tubular structure 21) underlying the multi-layered structure until the biodisintegrable coating has substantially or entirely disintegrated.

In some embodiments, one or more regions of a polyelectrolyte layer may be cross-linked to one or more regions of another polyelectrolyte layer (e.g., by selectively irradiating certain regions of the polyelectrolyte layers), while the polyelectrolyte layers may not be cross-linked to each other in other regions.

In some embodiments, a medical device can include a structure (e.g., a multi-layered structure) having a combination of cross-linked polyelectrolytes and biodisintegrable polyelectrolytes, to provide further tailoring of the disintegration of the device.

In certain embodiments, layers 27 and/or 30 can include one or more polyoxometalates, anions that can be used to provide a negative charge to a layer. In some embodiments, the presence of polyoxometalates in layers 27 and/or 30 can enhance the MRI visibility of material in lumen 23 of tubular structure 21 of stent 20. In certain embodiments, the presence of polyoxometalates in layers 27 and/or 30 can enhance the X-ray visibility of tubular structure 21 of stent 20. Polyoxometalates are nanosized inorganic oxygen clusters, such as inorganic metal oxygen clusters, that can be strictly uniform at the atomic level. The clusters are discrete molecular units with well-defined molecular formulas, in contrast to nanosized particles having infinite, extended lattice structures. Depending on the number of different types of non-oxygen atoms, polyoxometalates can be classified as isopolyanions or heteropolyanions. Isopolyanions can be described by the formula [MₓO_{y}]^{-p}; and heteropolyanions can be described by the formula [XₐM_{b}O_{c}]^{-q}, where M and X are different metals. Examples of metals include gadolinium, dysprosium, molybdenum, tungsten, vanadium, niobium, tantalum, cobalt, iron, ruthenium, titanium, nickel, chromium, platinum, zirconium, iridium, silicon, and boron. In some embodiments, the polyoxometalates are magnetic, for example, ferromagnetic, diamagnetic (e.g., including copper or bismuth), paramagnetic, or superparamagnetic. Examples of polyoxometalates include [Co₄(H₂O)₂(PW₉O₃₄)₂]¹⁰⁻; [Co₄(H₂O)₂(P₂W₁₅O₅₆)₂]¹⁶⁻; Keggin POMs (such as [XM₁₂O₄₀]^{3-/4-}, where X can be P or Si, and M can be W, Mo, Fe, Mn, Cr, Ni, or Ru); Preyssler POMs (such as [M(H₂O)P₅W₃₀O₁₁₀]^{14/12-}, where M can be Na or Eu); Lindqvist POMs (such as [M₆O₁₉]²⁻); and Anderson POMs (such as [XM₆O₂₄]^{m-}). Other examples of polyoxometalates are described, for example, in Casan-Pastor et al., Frontiers in Bioscience 9, 1759-1770, May 1, 2004; Clemente-Leon et al., Adv. Mater. 2001, 13, No. 8, April 18, 574-577; Liu et al., Journal of Cluster Science, Vol. 14, No. 3, September 2003, 405-419; Hu Changwen et al., "Polyoxometalate-based Organic-inorganic Hybrid Materials" Chemical Journal on Internet, Vol. 3, No. 6, page 22 (June 1, 2001); Kurth et al., Chem. Mater., 2000, 12, 2829-2831; and the entire issue of Chem. Rev. 1998, 98, 1. One or more layers 27 can include one type of molecular magnetic cluster or different types of clusters. Polyoxometalates also are described, for example, in U.S. Patent Publication No. US 2006 100 696.

Layers 27 can be assembled with layers 30 using a layer-by-layer technique in which the layers electrostatically self-assemble. In the layer-by-layer technique, a first layer having a first surface charge is deposited on an underlying substrate, followed by a second layer having a second surface charge that is opposite in sign to the surface charge of the first layer. Thus, the charge on the outer layer is reversed upon deposition of each sequential layer. Additional first and second layers can then be alternatingly deposited on the substrate to build multi-layered structure 26 to a predetermined or targeted thickness. The layer-by-layer technique allows structure 26 to be formed on tubular structure 21 directly and/or, for example, on a flexible sleeve (e.g., a polymer sleeve) carried by the tubular structure. As a result, structure 26 is capable of controlling the disintegration of tubular structure 21, while allowing stent 20 to remain flexible and adaptable to the vessel in which stent 20 is implanted.

Layers 27 may have the same thickness or different thicknesses. In some embodiments, the thickness of a layer may depend on the molecular weight of the polyelectrolyte(s) included in the layer, and/or the presence of other materials (e.g., nanoparticles) in the layer. For example, a layer including a relatively low molecular weight polyelectrolyte, such as low molecular weight heparin (e.g., heparin having a molecular weight of from 1,000 Daltons to 10,000 Daltons) may relatively thin. In certain embodiments, the thickness of a layer may depend on the conditions (e.g., salt concentration and/or pH) during the deposition of the layer. In some embodiments, an individual layer 27 and/or an individual layer 30 may have a thickness of at least 0.2 nanometer (e.g., at least 0.5 nanometer, at least one nanometer, at least five nanometers, at least 10 nanometers, at least 50 nanometers, at least 100 nanometers, at least 300 nanometers), and/or at most 500 nanometers (e.g., at most 300 nanometers, at most 100 nanometers, at most 50 nanometers, at most 10 nanometers, at most five nanometers, at most one nanometer, at most 0.5 nanometer).

As described above, tubular structure 21 of stent 20 is formed of a biodisintegrable material, such as a biodisintegrable metal, a biodisintegrable metal alloy, or a biodisintegrable non-metal. Examples of biodisintegrable metals include alkali metals, alkaline earth metals (e.g., magnesium), iron, zinc, aluminum. In some embodiments, a biodisintegrable metal can be sintered. Examples of biodisintegrable metal alloys include alkali metal alloys, alkaline earth metal alloys (e.g., magnesium alloys), iron alloys (e.g., alloys including iron and up to seven percent carbon), zinc alloys, and aluminum alloys. Examples of biodisintegrable non-metals include biodisintegrable polymers, such as polyiminocarbonates, polycarbonates, polyarylates, polylactides, or polyglycolic esters.

In certain embodiments, a biodisintegrable material can include at least one metallic component and at least one non-metallic component, or at least two different metallic components. In some embodiments, a biodisintegrable material can include at least one of the following: manganese, cobalt, nickel, chromium, copper, cadmium, lead, tin, thorium, zirconium, silver, gold, palladium, platinum, rhenium, silicon, calcium, lithium, aluminum, zinc, iron, carbon, and sulfur. In certain embodiments, a biodisintegrable material can include magnesium, titanium, zirconium, niobium, tantalum, zinc, or silicon, and lithium, sodium, potassium, calcium, iron, or manganese.

In certain embodiments, a medical device that is formed of one or more biodisintegrable materials can disintegrate at a rate of at least 0.2 micron per day (e.g., at least 0.5 micron per day, at least one micron per day, at least two microns per day, at least three microns per day, at least four microns per day) and/or at most five microns per day (e.g., at most four microns per day, at most three microns per day, at most two microns per day; at most one micron per day, at most 0.5 micron per day). In some embodiments, a medical device that is formed of one or more biodisintegrable materials can disintegrate over a period of at least five days (e.g., at least one week, at least two weeks, at least three weeks, at least four weeks, at least six weeks, at least eight weeks, at least 12 weeks, at least 16 weeks, at least 20 weeks, at least 24 weeks, at least 12 months).

Biodisintegrable materials are described, for example, in Bolz, U.S. Patent No. 6,287,332; Heublein, U.S. Patent Application Publication No. US 2002/0004060 A1; Kohn et al., U.S. Patent No. 5,587,507; and Kohn et al., U.S. Patent No. 6,475,477.

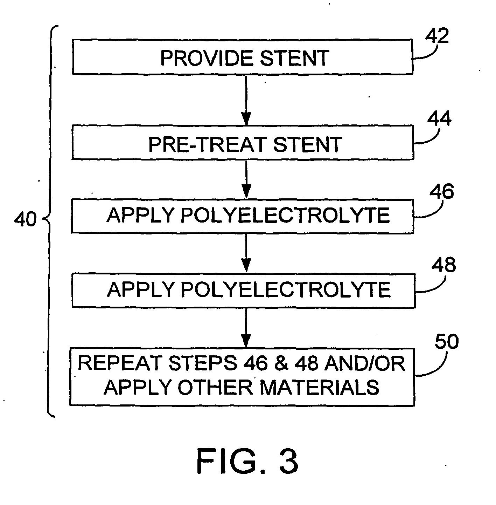

Referring to FIG. 3, an embodiment of a method 40 of making stent 20 using a layer-by-layer technique is shown. Method 40 includes providing a starting stent (step 42) and pretreating the starting stent for layer-by-layer deposition (step 44). Next, a charged layer 27 containing a polyelectrolyte can be applied on the starting stent (step 46). A charged layer 30 containing a polyelectrolyte can then be applied to the previously applied charged layer 27 (step 48). Steps 46 and 48 can then repeated to build a multi-layered structure 26 of a desired thickness to form stent 20. In some embodiments, as described below, multi-layered structure 26 can further include one or more layers including a therapeutic agent, one or more layers including a radiopaque material, and/or one or more layers capable of enhancing the mechanical properties of structure 26. These additional layers can be applied between layers 27 and/or layers 30, in any combination. Layer-by-layer self-assembly is described, for example, in Liu et al., Journal of Cluster Science, Vol. 14, No. 3, September 2003, 405-419; and Caruso et al., Langmuir 1998, 14, 3462-3465.

The starting stent that is provided according to step 42 can be manufactured, or the starting stent can be obtained commercially. Methods of making stents are described, for example, in Saunders, U.S. Patent No. 5,780,807, and Stinson, U.S. Application Publication US 2004/0000046 A1. Stents are also available, for example, from Boston Scientific Corporation.

The provided stent can be formed of any biocompatible, biodisintegrable material, such as the materials described above with reference to tubular structure 21 of stent 20. The biocompatible material can be suitable for use in, for example, a balloon-expandable stent.

After the stent has been provided, the stent can be pretreated (step 44). For example, the stent can be cleaned to remove surface contaminants, such as oil, that can affect the homogeneity to which multi-layered structure 26 can be formed. The stent can be cleaned, for example, in a mixture such as acetone, H₂O₂/HCl, HCl/HNO₃, H₂SO₄/K₂Cr₂O₇, H₂O₂/NH₃, and/or NaOH/NaOCl. The stent can also be pretreated with a solution including 10⁻² M SDS/0.12 N HCl for 15 minutes at 100°C.

Next, while certain stent materials may be inherently charged and thus lend themselves to layer-by-layer assembly, in certain embodiments (e.g., embodiments in which the stent does not have an inherent net surface charge), a surface charge may be provided to the stent. For example, where the stent to be coated is conductive, a surface charge can be provided by applying an electrical potential to the stent. Once a first polyelectrolyte layer is applied in this fashion, a second polyelectrolyte layer having a second surface charge that is opposite in sign to the surface charge of the first polyelectrolyte layer can be applied, and so forth.

As another example, the stent can be provided with a positive charge by covalently attaching functional groups having a positive charge (e.g., amine, imine or other basic groups) to the stent, or can be provided with a negative charge by covalently attaching functional groups having a negative charge (e.g., carboxylic, phosphonic, phosphoric, sulfuric, sulfonic, or other acid groups) to the stent.

As an additional example, a surface charge can be provided by exposing the stent to a charged amphiphilic substance. Amphiphilic substances can include any substance having hydrophilic and hydrophobic groups. In some embodiments, the amphiphilic substance can include at least one electrically charged group that can provide the stent surface with a net electrical charge. In such embodiments, the amphiphilic substances can also be referred to as ionic amphiphilic-substances.

In certain embodiments, an amphiphilic polyelectrolyte can be used as an ionic amphiphilic substance. For example, a polyelectrolyte comprising charged groups (which are hydrophilic) as well as hydrophobic groups, such as polyethyleneimine (PEI) or poly(styrene sulfonate) (PSS), can be employed. Cationic and anionic surfactants can also be used as amphiphilic substances in some embodiments. Cationic surfactants include quaternary ammonium salts (R₄N⁺X⁻), in which R is an organic radical and X⁻ is a counter-anion (e.g., a halogenide), for example, didodecyldimethylammonium bromide (DDDAB), alkyltrimethylammonium bromides such as hexadecyltrimethylammonium bromide (HDTAB), dodecyltrimethylammonium bromide (DTMAB), myristyltrimethylammonium bromide (MTMAB), or palmityl trimethylammonium bromide, or N-alkylpyridinium salts, or tertiary amines (R₃NH⁺X⁻), for example, cholesteryl-3β-N-(dimethyl-aminoethyl)-carbamate or mixtures thereof. Anionic surfactants include alkyl or olefin sulfate (R-OSO₃M), for example, a dodecyl sulfate such as sodium dodecyl sulfate (SDS), a lauryl sulfate such as sodium lauryl sulfate (SLS), or an alkyl or olefin sulfonate (R-SO₃M), for example, sodium-n-dodecylbenzene sulfonate, or fatty acids (R-COOM), for example, dodecanoic acid sodium salt, or phosphoric acids or cholic acids or fluoro-organics, for example, lithium-3-[2-(perfluoroalkyl)ethylthio]propionate or mixtures thereof, where R is an organic radical and M is a counter-cation.

Thus, a surface charge can be provided on the stent by adsorbing cations (e.g., protamine sulfate, polyallylamine, polydiallyldimethylammonium species, polyethyleneimine, chitosan, gelatin, spermidine, and/or albumin) or by adsorbing anions (e.g., polyacrylic acid, sodium alginate, polystyrene sulfonate, eudragit, gelatin (an amphiphilic polymer that fits in both categories depending how it is being prepared), hyaluronic acid, carrageenan, chondroitin sulfate, and/or carboxymethylcellulose) to the surface of the stent as a first charged layer. As an example, polyethyleneimine (PEI, Aldrich, MW ∼25 kD) can be dissolved in water in a concentration of about 0.5 g/L to apply a first coating. In some embodiments, more than one surface charge layer can be applied to provide complete coverage of the stent. Application of surface charge layers is described, for example, in "Multilayer on Solid Planar Substrates", Multilayer Thin Films, Sequential Assembly of Nanocomposite Materials, Wiley-VCH ISBN 3-527-30440-1, Chapter 14; and "Surface-Chemistry Technology for Microfluidics", Hau, Winky L. W. et al., J. Micromech. Microeng. 13 (2003) 272-278.

The species for establishing a surface charge can be applied to the stent by a variety of techniques. Examples of techniques include spraying techniques, dipping techniques, roll and brush coating techniques, techniques involving coating via mechanical suspension such as air suspension, ink jet techniques, spin coating techniques, web coating techniques, microstamping techniques , and combinations of these processes. Microstamping techniques using polydimethylsiloxane (PDMS) technology are described, for example, in Lin et al., "Micropatterning proteins and cells on polylactic acid and poly(lactide-co-glycolide)", Biomaterials 26 (2005), 3655-3662. Dipping and spraying techniques (without masking) can be employed, for example, to apply the species to an entire stent. Roll coating, brush coating and ink jet printing can be employed, for example, to apply the species only to selected portions of the stent (e.g., in the form of a pattern).

Once a preselected charge is provided on the stent, the stent can be coated with a layer of an oppositely charged material. After each application of a layer, the stent can be washed to remove excess material. Multi-layer structure 26 can be formed by repeated treatment with alternating, oppositely charged materials, for example, by alternating treatment of a positive polyelectrolyte with treatment with a negative polyelectrolyte to build layers 27 and 30 (steps 46 and 48). Layers 27 and 30 self-assemble by electrostatic layer-by-layer deposition, thus forming multi-layered structure 26 over the stent.

In some embodiments, multi-layered structure 26 can include nanoparticles. The nanoparticles can, for example, enhance the mechanical properties (e.g., strength) of the structure. The nanoparticles can have at least one dimension (e.g., the thickness for a nanoplate, the diameter for a nanosphere, a nanocylinder and a nanotube) that is less than 1000 nanometers (e.g., less than 100 nanometers). Nanoplates can have at least one dimension that is less than 1000 nanometers; nanofibers can have at least two orthogonal dimensions (e.g., the diameter for a cylindrical nanofiber) that are less than 1000 nanometers; and other nanoparticles can have three orthogonal dimensions that are less than 1000 nanometers (e.g., the diameter for nanospheres).

Examples of nanoparticles include carbon, ceramic and metallic nanoparticles including nanoplates, nanotubes, and nanospheres, and other nanoparticles. Specific examples of nanoplates include synthetic or natural phyllosilicates including clays and micas (which may optionally be intercalated and/or exfoliated) such as montmorillonite, hectorite, hydrotalcite, vermiculite and laponite. Specific examples of nanotubes and nanofibers include single-wall and multi-wall carbon nanotubes, such as fullerene nanotubes, vapor grown carbon fibers, alumina nanofibers, titanium oxide nanofibers, tungsten oxide nanofibers, tantalum oxide nanofibers, zirconium oxide nanofibers, and silicate nanofibers such as aluminum silicate nanofibers. Other examples of nanoparticles (e. g., nanoparticles having three orthogonal dimensions that are less than 1000 nm) include fullerenes (e.g., bucky balls), silica nanoparticles, aluminum oxide nanoparticles, titanium oxide nanoparticles, tungsten oxide nanoparticles, tantalum oxide nanoparticles, zirconium oxide nanoparticles, dendrimers, and monomeric silicates such as polyhedral oligomeric silsequioxanes (POSS), including various functionalized POSS and polymerized POSS. The carbon nanotubes and carbon nanofibers can have a diameter ranging from 0.5 nm to 200 nm. Still other examples of nanoparticles include compositions of the Mo-S-I family, such as Mo₆S₃I₆ and Mo₃S₆I, which are described in Vrbani 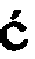 et al., Nanotechnology 15 (2004) 635-638; Rem 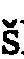 kar et al., *Science* 292 (2001) 479; and Mihailovi 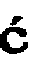 et al., Phys. Rev. Lett. 90 (2003) 146401-1. Mo₃S₆I, for example when doped, can exhibit a large paramagnetic susceptibility, which can further enhance the magnetic shielding capability of the multi-layered structure.

In certain embodiments, a polyelectrolyte layer can be used to encapsulate one or more nanoparticles, such as magnetic nanoparticles (e.g., CoFe₂O₄) and those described herein. Polymer/magnetic nanoparticles systems are described, for example, in Sing et al., Electrochimica Acta 49 (2004) 4605-4612. The polymer/nanoparticles composite can, for example, enhance the mechanical properties of the structure and/or enhance a magnetic shielding effect.

Various techniques can be used to provide charges on nanoparticles that are not inherently charged. For example, a surface charge can be provided by adsorbing or otherwise attaching species on the nanoparticles that have a net positive or negative charge, for example, charged amphiphilic substance such as amphiphilic polyelectrolytes and cationic and anionic surfactants (see above). Where the nanoparticles are sufficiently stable, surface charges can sometimes be established by exposure to highly acidic conditions. For example, carbon nanoparticles, such as carbon nanotubes, can be partially oxidized by refluxing in strong acid to form carboxylic acid groups (which ionize to become negatively charged carboxyl groups) on the nanoparticles. Establishing a surface charge on nanoparticles can also provide a relatively stable and uniform suspension of the nanoparticles, due at least in part to electrostatic stabilization effects. Layer-by-layer assembly to form alternating layers of SWNT and polymeric material have also been described, for example, in Arif A. Mamedov et al., "Molecular Design of Strong Singlewall Carbon Nanotube/Polyelectrolyte Multilayer Composites", Nature Material, Vol. 1, No. 3, 2002, pages 191-194. The nanoparticles can be positively charged or negatively charged, and both types of nanoparticles can be used in a medical device. A medical device can include one composition of nanoparticles or different compositions of nanoparticles.

As indicated above, in some embodiments, multi-layered structure 26 can further include one or more layers including a therapeutic agent, one or more layers including a radiopaque material, and/or one or more layers capable of enhancing the mechanical properties of structure 26.

As an example, in some embodiments, a medical device can include one or more non-polyelectrolyte layers that include one or more therapeutic agents. The non-polyelectrolyte layers can be biodisintegrable or biostable. Examples of non-polyelectrolyte biodisintegrable materials include polylactides (PLA), polyglycolides (PGA), poly(lactide-co-glycolides) (PLGA), polyanhydrides, and polyorthoesters. For example, a stent can include a tubular structure and a multi-layered structure formed of polyelectrolytes located on the tubular structure. The stent can further include one or more non-polyelectrolyte layers, which can be added to the stent using, for example, spraying techniques. The non-polyelectrolyte layer(s) may be added on top of the multi-layered structure, and/or underneath the multi-layered structure. In some embodiments, a stent can include more than one multi-layered structure, and/or can include one or more non-polyelectrolyte layers located between the multi-layered structures. In some embodiments, the non-polyelectrolyte layer(s) may be thicker than one or more of the layers in the multi-layered structure(s). In certain embodiments, a non-polyelectrolyte layer may exhibit better adhesion to the surface of a tubular structure of a stent than a polyelectrolyte layer.

As another example, one or more therapeutic agents can be disposed on or within multi-layered structure 26 giving the medical device, for example, a drug releasing function upon implantation. The therapeutic agent can be charged, for example, because it is itself a charged molecule or because it is intimately associated with a charged molecule. Examples of charged therapeutic agents include small molecule and polymeric therapeutic agents containing ionically dissociable groups. In some embodiments in which the therapeutic agent does not possess one or more charged groups, it can nevertheless be provided with a charge, for example, through non-covalent association with a charged species. Examples of non-covalent associations include hydrogen bonding, and hydrophilic/lipophilic interactions. For instance, the therapeutic agent can be associated with an ionic amphiphilic substance.

In certain embodiments in which a charged therapeutic agent is used, one or more layers of the charged therapeutic agent can be deposited during the course of assembling multi-layer structure 26. For example, the therapeutic agent can be a polyelectrolyte (e.g., where the therapeutic agent is a polypeptide or a polynucleotide) and can be used to create one or more polyelectrolyte layers within multi-layer structure 26. In other embodiments, the charged therapeutic agent is not a polyelectrolyte (e.g., it may be a charged small molecule drug), but one or more layers of the charged therapeutic agent can be substituted for one or more layers of the same charge (i.e., positive or negative) during the layer-by-layer assembly process.

In still other embodiments, the therapeutic agent can provided within charged nanocapsules, which are formed, for example, using layer-by-layer techniques such as those described herein and in commonly assigned U.S. Patent Publication No. US 2005/129727. In these embodiments, one or more layers of the charged nanocapsules can be deposited during the course of the layer-by-layer assembly process.

In still other embodiments, multi-layer structure 26 is loaded with a therapeutic agent subsequent to its formation. For example, the porosity, and thus the permeability, of structure 26 can be modified by modifying the pH exposed to the structure, as described, for example, in Antipov, A. A. et al., "Polyelectrolyte Multilayer Capsule Permeability Control", Colloids and Surfaces A: Physicochemical and Engineering Aspects, 198-200 (2002) pp. 535-541.

Examples of therapeutic agents and methods of incorporated the agents are described in U.S. Patent Publication No. US 2005/261760, and Kunz et al., U. S. Patent No. 5,733,925. Some examples of therapeutic agents include non-genetic therapeutic agents, genetic therapeutic agents, vectors for delivery of genetic therapeutic agents, cells, and therapeutic agents identified as candidates for vascular treatment regimens, for example, as agents targeting restenosis.

To enhance the radiopacity of stent 20, a radiopaque material, such as gold nanoparticles, can be incorporated into multi-layered structure 26. As an example, gold nanoparticles can be made positively charged by applying a outer layer of lysine to the nanoparticles, as described, for example, in "DNA Mediated Electrostatic Assembly of Gold Nanoparticles into Linear Arrays by a Simple Dropcoating Procedure", Murali Sastrya and Ashavani Kumar, Applied Physics Letters, Vol. 78, No. 19, 7 May 2001. Other radiopaque materials include, for example, tantalum, platinum, palladium, tungsten, iridium, and their alloys.

The layer-by-layer assembly can be conducted by exposing a selected charged substrate (e.g., a stent) to solutions or suspensions that contain species of alternating net charge, including solutions or suspensions that contain charged magnetic clusters, charged polyelectrolytes, and, optionally, charged therapeutic agents and/or nanoparticles. The concentration of the charged species within these solutions and suspensions, which can be dependent on the types of species being deposited, can range, for example, from 0.01 mg/ml to 30 mg/ml. The pH of these suspensions and solutions can be such that the magnetic clusters, polyelectrolytes, and optional therapeutic agents and/or nanoparticles maintain their charge. Buffer systems can be used to maintain charge.

The solutions and suspensions containing the charged species (e.g., solutions/suspensions of magnetic clusters, polyelectrolytes, or other optional charged species such as charged therapeutic agents and/or charged nanoparticles) can be applied to the charged substrate surface using a variety of techniques. Examples of techniques include spraying techniques, dipping techniques, roll and brush coating techniques, techniques involving coating via mechanical suspension such as air suspension, ink jet techniques, spin coating techniques, web coating techniques, microstamping techniques, and combinations of these processes. Layers can be applied over an underlying substrate by immersing the entire substrate (e.g., stent) into a solution or suspension containing the charged species, or by immersing half of the substrate into the solution or suspension, flipping the same, and immersing the other half of the substrate into the solution or suspension to complete the coating. In some embodiments, the substrate is rinsed after application of each charged species layer, for example, using a washing solution with a pH that maintains the charge of the outer layer.

Using the techniques described herein, multiple layers of alternating charge can be applied over the underlying substrate, including the application of one or more charged layers 27 and the application of one or more charged layers 30. The number of each layer and/or the total thickness of multi-layered structure 26 can be determined empirically and can be a function of, for example, the compositions of the layers and the type of medical device. For example, for a given medical device, the number of layers, their sequences and compositions, and/or the total thickness of multi-layered structure 26 can be varied and the effectiveness of the multi-layered structure can be tested. After an effective combination is determined, the same combination can be repeated. In some embodiments, at least 10 layers (e.g., at least 20 layers, at least 30 layers, at least 50 layers, at least 100 layers, at least 200 layers) and/or at most 300 layers (e.g., at most 200 layers, at most 100 layers, at most 50 layers, at most 30 layers, at most 20 layers) can be applied over the substrate. The total thickness of multi-layered structure 26 can be a function of the materials (e.g., polyelectrolytes) used. In some embodiments, the total thickness of multi-layered structure 26 can be at least five nanometers (e.g., at least 10 nanometers; at least 50 nanometers; at least 100 nanometers; at least 500 nanometers; at least 1000 nanometers; at least 1500 nanometers; at least 2000 nanometers; at least 5000 nanometers; at least 10,000 nanometers; at least 20,000 nanometers; at least 30,000 nanometers) and/or at most 40,000 nanometers (e.g., at most 30,000 nanometers; at most 20,000 nanometers; at most 10,000 nanometers; at most 5000 nanometers; at most 2000 nanometers; at most 1500 nanometers; at most 1000 nanometers, at most 500 nanometers, at most 100 nanometers, at most 50 nanometers, at most 10 nanometers).

In use, stent 20 can be delivered and expanded using, for example, a balloon catheter system or other stent delivery systems. Catheter systems are described in, for example, Wang U.S. 5,195,969, and Hamlin U.S. 5,270,086. Stents and stent delivery are also exemplified by the Radius® or Symbiot® systems, available from Boston Scientific Scimed, Maple Grove, MN.

Stent 20 can be of any desired shape and size (e.g., coronary stents, aortic stents, peripheral vascular stents, gastrointestinal stents, urology stents, cerebral stents, urethral stents, ureteral stents, biliary stents, tracheal stents, esophageal stents, neurology stents). Depending on the application, stent 20 can have a diameter of between, for example, one millimeter to 46 millimeters. In certain embodiments, a coronary stent can have an expanded diameter of from about two millimeters to about six millimeters. In some embodiments, a peripheral stent can have an expanded diameter of from about four millimeters to about 24 millimeters. In certain embodiments, a gastrointestinal and/or urology stent can have an expanded diameter of from about six millimeters to about 30 millimeters. In some embodiments, a neurology stent can have an expanded diameter of from about one millimeter to about 12 millimeters. An abdominal aortic aneurysm (AAA) stent and a thoracic aortic aneurysm (TAA) stent can have a diameter from about 20 millimeters to about 46 millimeters. Stent 20 can be balloon-expandable, self-expandable, or a combination of both (e.g., Andersen et al., U.S. Patent No. 5,366,504).

While a number of embodiments have been described above, the invention is not so limited.

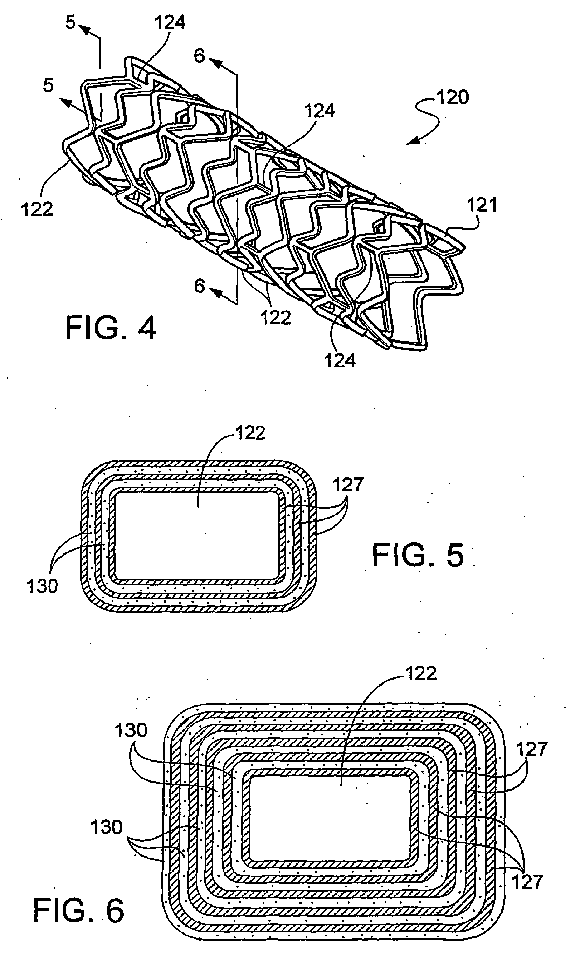

As an example, in certain embodiments, one portion of a medical device can have a different number of layers on it than another portion of a medical device. For example, FIG. 4 shows a stent 120 in the form of a biodisintegrable tubular structure 121 defined by a plurality of bands 122 and a plurality of connectors 124 extending between and connecting adjacent bands. As shown in FIG. 5, one portion of stent 120 (as shown, a portion of a band 122) is coated with a multi-layered structure 126 including a total of five biodisintegrable layers (three charged layers 127 and two charged layers 130). As shown in FIG. 6, another portion of stent 120 (as shown, a portion of another band 122) is coated with a multi-layered structure 150 including a total of 10 biodisintegrable layers (five charged layers 127 and five charged layers 130). Thus, during use, multi-layered structure 126 will tend to disintegrate more quickly than multi-layered structure 150. As a result, the portion of tubular structure 121 that is underneath multi-layered structure 126 will tend to disintegrate more quickly than the portion of tubular structure 121 that is underneath multi-layered structure 150.

While a medical device including five layers on one portion and 10 layers on another portion has been shown, other embodiments are possible. For example, in some embodiments, one portion of a medical device can include a multi-layered structure with at least 10 layers (e.g., at least 20 layers, at least 30 layers, at least 40 layers), and another portion of a medical device can include a multi-layered structure with at least 20 layers (e.g., at least 30 layers, at least 40 layers, at least 50 layers). For example, one portion of a medical device may include a multi-layered structure with 10 layers and another portion of the medical device may include a multi-layered structure with 40 layers. In certain embodiments, a multi-layered structure on one portion of a medical device can include from five to 50 more layers (e.g., from 10 to 30 more layers) than a multi-layered structure on another portion of the medical device.

In some embodiments, one or more portions of a medical device may not include any polyelectrolyte layers or may not include any layers at all.

In some embodiments, stents having portions with different numbers of layers on them can be formed by dipping one end (e.g., 2/3) of a stent in one material, turning the stent around, and dipping the other end (e.g., 2/3) of the stent in another material, and repeating the process multiple times. The result is that the middle of the stent (e.g., the middle 1/3 of the stent) receives more layers than either end of the stent. In certain embodiments, stents having portions with different numbers of layers on them can be formed by other techniques, such as ink jet techniques, microstamping, spraying, roll coating, or brush coating.

As another example, while a stent including a tubular structure that is formed entirely of a biodisintegrable material has been described, in some embodiments, the tubular structure of a stent can include one or more biostable materials, in addition to including one or more biodisintegrable materials. For example, the tubular structure of a stent may include biostable regions and biodisintegrable regions that are designed to biodisintegrate a certain period of time after the stent has been delivered to a target site. In certain embodiments, the disintegration of the biodisintegrable regions may enhance the MRI-compatibility of the stent. One or more polyelectrolytes may be used (as described above) to control the disintegration of one or more of the biodisintegrable regions of the stent. The polyelectrolytes may be in the form of layers over the biodisintegrable and/or biostable regions of the stent. Examples of biostable materials include Nitinol (e.g., 55% nickel, 45% titanium) and stainless steel. Specific examples of biocompatible materials are described, for example, in Weber et al., U.S. Patent Application Publication No. US 2004/0230290 A1, published on November 18; 2004; Craig et al., U.S. Patent Application Publication No. US 2003/0018380 A1, published on January 23, 2003; Craig et al., U.S. Patent Application Publication No. US 2002/0144757 A1, published on October 10, 2002; and Craig et al., U.S. Patent Application Publication No. US 2003/0077200 A1 published on April 24, 2003. Stents including biostable and biodisintegrable regions are described, for example, in U.S. Patent Publication No. US 2006/122694.

As a further example, in certain embodiments, a multi-layered structure may include at least two positively charged layers that are formed of different materials (e.g., different polyelectrolytes) and/or at least two negatively charged layers that are formed of different materials (e.g., different polyelectrolytes).

As an additional example, in some embodiments, one portion of a medical device may be coated with a multi-layered structure, while another portion of the medical device may not have any coatings on it, or may be coated with just one layer.

As another example, in some embodiments, a multi-layered structure such as multi-layered structure 26 can be formed on a substrate, removed from the substrate, and subsequently applied (e.g., with an adhesive) to a medical device. The substrate can be removed by destroying it, for example, by melting, sublimation, combustion, and/or dissolution, to free multi-layered structure 26 For example, a removable substrate made of dental waxes (such as those available from MDL Dental Products, Inc., Seattle, WA, USA) or polyvinyl alcohol (PVOH) can be used. These materials can melt at moderately elevated temperatures (e.g., 60°C) and dissolve in hot water, respectively. Other methods of using a removable substrate are described in Sukhorukov et al., "Comparative Analysis of Hollow and Filled Polyelectrolyte Microcapsules Templated on Melamine Formaldehyde and Carbonate Cores, Macromol.", Chem. Phys., 205, 2004, pp. 530-535; and U.S. Patent Publication No. US 2005/261760.

As an additional example, one or more reinforcement aids can be provided adjacent to, and/or within, a multi-layered structure such as multi-layered structure 26 to enhance its mechanical properties. For example, one or more reinforcement aids can be applied to a substrate, followed by a series of polyelectrolyte layers. As another example, a first series of polyelectrolyte layers can be provided, followed by the application of one or more reinforcement aids, followed by a second series of polyelectrolyte layers. Examples of reinforcement aids include fibrous reinforcement members such as metal fiber meshes, metal fiber braids, metal fiber windings, intermingled fibers (e.g., metal fibers, carbon fibers, high density polyethylene fibers, liquid polymer crystals) and so forth. The reinforcement aids can be provided with a surface charge to enhance incorporation of the reinforcement aids onto or into multi-layered structure 26. For example, layer-by-layer techniques can be used to encapsulate the reinforcement aids, thereby providing them with a charged outer layer and enhancing interaction of the reinforcement aids with an adjacent layer (e.g., a polyelectrolyte layer) of opposite charge. The loading of the reinforcement aids can be such that they form a conductive loop or solenoid.

As another example, in certain embodiments, a stent such as stent 20 can also be a part of a stent-graft or a covered stent. In other embodiments, a stent such as stent 20 can include and/or be attached to a biocompatible, non-porous or semi-porous polymer matrix made of polytetrafluoroethylene (PTFE), expanded PTFE, polyethylene, urethane, or polypropylene.

As a further example, in some embodiments, a multi-layered structure such as multi-layered structure 26 can be applied to other types of medical devices. For example, multi-layered structure 26 can be applied to grafts, filterwires, valves, filters (e.g., vena cava filters), distal protection devices, guidewires, and other implantable devices. In some embodiments, a multi-layered structure such as multi-layered structure 26 can be applied to a catheter (e.g., a renal or vascular catheter such as a balloon catheter). In certain embodiments, a multi-layered structure such as multi-layered structure 26 can be applied to a balloon. In some embodiments, a multi-layered structure such as multi-layered structure 26 can be applied to a coil (e.g., an aneurysm coil). Coils are described, for example, in Twyford, Jr. et al., U.S. Patent No. 5,304,195.

As an additional example, in certain embodiments, a biodisintegrable layer (e.g., a biodisintegrable non-polyelectrolyte layer) on a medical device can be coated with one or more other layers, such as one or more polyelectrolyte layers and/or one or more biodisintegrable layers.

## Claims

1. A medical device, comprising:
a tubular body comprising a biodisintegrable material; and
a first polyelectrolyte on the tubular body.

2. The medical device of claim 1, wherein the first polyelectrolyte is biodisintegrable.

3. The medical device of claim 1, wherein the first polyelectrolyte comprises a polyacid.

4. The medical device of claim 3, wherein the polyacid is selected from the group consisting of polyphosphoric acids, polyvinylsulfuric acids, polyvinylsulfonic acids, polyvinylphosphonic acids, and polyacrylic acids.

5. The medical device of claim 1, wherein the first polyelectrolyte comprises a polybase.

6. The medical device of claim 5, wherein the polybase comprises polyallylamine, polyethylimine, polyvinylamine, and polyvinylpyridine.

7. The medical device of claim 1, wherein the first polyelectrolyte comprises a polycation.

8. The medical device of claim 7, wherein the polycation is selected from the group consisting of poly(allylamine) polycations, polyethyleneimine polycations, polydiallyldimethylammonium polycations, protamine sulfate polycations, chitosan polycations, gelatin polycations, spermidine polycations, poly(*N*-octyl-4-vinyl pyridinium iodide) polycations, and albumin polycations.

9. The medical device of claim 1, wherein the first polyelectrolyte comprises a polyanion.

10. The medical device of claim 9, wherein the polyanion is selected from the group consisting of poly(styrene sulfonate) polyanions, polyacrylic acid polyanions, sodium alginate polyanions, eudragit polyanions, gelatin polyanions, hyaluronic acid polyanions, carrageenan polyanions, chondroitin sulfate polyanions, and carboxymethylcellulose polyanions.

11. The medical device of claim 1, wherein the first polyelectrolyte comprises a polymer selected from the group consisting of heparin, proteins, polyglycolic acid, polylactic acid, polyamides, poly-2-hydroxy-butyrate, polycaprolactone, and poly(lactic-co-glycolic)acid.

12. The medical device of claim 1, wherein the biodisintegrable material comprises a metal.

13. The medical device of claim 1, wherein the biodisintegrable material comprises a metal selected from the group consisting of alkali metals, alkaline earth metals, iron, zinc, and aluminum.

14. The medical device of claim 1, wherein the biodisintegrable material comprises iron.

15. The medical device of claim 1, wherein the biodisintegrable material comprises magnesium.

16. The medical device of claim 1, wherein the biodisintegrable material comprises a metal alloy.

17. The medical device of claim 1, wherein the biodisintegrable material comprises an iron alloy.

18. The medical device of claim 1, wherein the biodisintegrable material comprises a magnesium alloy.

19. The medical device of claim 1, wherein the biodisintegrable material comprises a first component selected from the group consisting of magnesium, titanium, zirconium, niobium, tantalum, zinc, and silicon, and a second component selected from the group consisting of lithium, sodium, potassium, calcium, iron, and manganese.

20. The medical device of claim 1, wherein the biodisintegrable material comprises a non-metal.

21. The medical device of claim 1, wherein the biodisintegrable material comprises a polymer.

22. The medical device of claim 1, wherein the medical device comprises a first layer comprising the first polyelectrolyte, the first layer being supported by the tubular body.

23. The medical device of claim 22, wherein the first layer has a thickness of at most 500 nanometers.

24. The medical device of claim 23, wherein the first layer has a thickness of at least 0.2 nanometer.

25. The medical device of claim 22, wherein the first layer defines at least one opening.

26. The medical device of claim 22, wherein the first layer contacts the tubular body.

27. The medical device of claim 22, further comprising a second layer that is supported by the tubular body.

28. The medical device of claim 27, wherein the second layer contacts the first layer.

29. The medical device of claim 27, wherein the medical device comprises a plurality of first layers and a plurality of second layers.

30. The medical device of claim 29, wherein at least two of the first layers are spaced from each other by one or more second layers.

31. The medical device of claim 27, wherein the second layer comprises a second polyelectrolyte.

32. The medical device of claim 31, wherein the second polyelectrolyte is different from the first polyelectrolyte.

33. The medical device of claim 31, wherein the first polyelectrolyte is positively charged and the second polyelectrolyte is negatively charged.

34. The medical device of claim 1, wherein the medical device further comprises a second polyelectrolyte on the tubular body.

35. The medical device of claim 34, wherein the first polyelectrolyte and the second polyelectrolyte are cross-linked to each other.

36. The medical device of claim 35, wherein the first polyelectrolyte and the second polyelectrolyte are cross-linked to each other by at least one covalent bond.

37. The medical device of claim 1, wherein the medical device comprises a plurality of layers.

38. The medical device of claim 37, wherein a first portion of the medical device comprises a greater number of layers than a second portion of the medical device.

39. The medical device of claim 38, wherein the first portion includes at least 20 layers.

40. The medical device of claim 39, wherein the second portion includes at least 10 layers.

41. The medical device of claim 37, wherein the plurality of layers has a total thickness of at most 1500 nanometers.

42. The medical device of claim 41, wherein the plurality of layers has a total thickness of at least five nanometers.

43. The medical device of claim 37, wherein the medical device comprises at least 10 layers.

44. The medical device of claim 43, wherein the medical device comprises at most 300 layers.

45. The medical device of claim 1, wherein the medical device comprises an endoprosthesis.

## Patentansprüche

1. Medizinische Vorrichtung, die folgendes aufweist:
einen rohrförmigen Körper, der ein nicht biologisch abbaubares Material aufweist; und
einen ersten Polyelektrolyt auf diesem rohrförmigen Körper.

2. Medizinische Vorrichtung nach Anspruch 1,
wobei der erste Polyelektrolyt nicht biologisch abbaubar ist.

3. Medizinische Vorrichtung nach Anspruch 1,
wobei der erste Polyelektrolyt eine Polysäure aufweist.

4. Medizinische Vorrichtung nach Anspruch 3,
wobei die Polysäure aus der Gruppe ausgewählt ist, die aus Polyphosphorsäuren, Polyvinylschwefelsäuren, Polyvinylsulfonsäuren, Polyvinylphosponsäuren und Polyacrylsäuren besteht.

5. Medizinische Vorrichtung nach Anspruch 1,
wobei der erste Polyelektrolyt eine Polybase aufweist.

6. Medizinische Vorrichtung nach Anspruch 5,
wobei die Polybase Polyallylamin, Polyethylimin, Polyvinylamin und Polyvinylpyridin aufweist.

7. Medizinische Vorrichtung nach Anspruch 1,
wobei der erste Polyelektrolyt ein Polykation aufweist.

8. Medizinische Vorrichtung nach Anspruch 7,
wobei das Polykation aus der Gruppe ausgewählt ist, die aus Poly(allylamin)-Polykationen, Polyethylenimin-Polykationen, Polydiallyldimethylammonium-Polykationen, Protaminsulfat-Polykationen, Chitosan-Polykationen, Gelatine-Polykationen, Spermidin-Polykationen, Poly(N-octyl-4-vinylpyridiniumiodid)-Polykationen und Albumin-Polykationen besteht.

9. Medizinische Vorrichtung nach Anspruch 1,
wobei der erste Polyelektrolyt ein Polyanion aufweist.

10. Medizinische Vorrichtung nach Anspruch 9,
wobei das Polyanion aus der Gruppe ausgewählt ist, die aus Poly(styrolsulfonat)-Polyanionen, Polyacrylsäure-Polyanionen, Natriumalginat-Polyanionen, Eudragit-Polyanionen, Gelatine-Polyanionen, Hyaluronsäure-Polyanionen, Carrageen-Polyanionen, Chondroitinsulfat-Polyanionen und Carboxymethylcellulose-Polyanionen besteht.

11. Medizinische Vorrichtung nach Anspruch 1,
wobei der erste Polyelektrolyt ein Polymer aufweist, das aus der Gruppe ausgewählt ist, die aus Heparin, Proteinen, Polyglycolsäure, Polymilchsäure, Polyamiden, Poly-2-hydroxybutyrat, Polycaprolacton und Poly(milch-co-glycol)säure besteht.

12. Medizinische Vorrichtung nach Anspruch 1,
wobei das nicht biologisch abbaubare Material ein Metall aufweist.

13. Medizinische Vorrichtung nach Anspruch 1,
wobei das nicht biologisch abbaubare Material ein Metall aus der Gruppe ausgewählt ist, die aus Alkalimetallen, Erdalkalimetallen, Eisen, Zink und Aluminium besteht.

14. Medizinische Vorrichtung nach Anspruch 1,
wobei das nicht biologisch abbaubare Material Eisen aufweist.

15. Medizinische Vorrichtung nach Anspruch 1,
wobei das nicht biologisch abbaubare Material Magnesium aufweist.

16. Medizinische Vorrichtung nach Anspruch 1,
wobei das nicht biologisch abbaubare Material eine Metallegierung aufweist.

17. Medizinische Vorrichtung nach Anspruch 1,
wobei das nicht biologisch abbaubare Material eine Eisenlegierung aufweist.

18. Medizinische Vorrichtung nach Anspruch 1,
wobei das nicht biologisch abbaubare Material eine Magnesiumlegierung aufweist.

19. Medizinische Vorrichtung nach Anspruch 1,
wobei das nicht biologisch abbaubare Material eine erste Komponente, die aus der Gruppe ausgewählt ist, die aus Magnesium, Titan, Zirconium, Niob, Tantal, Zink und Silicium besteht, und eine zweite Komponente aufweist, die aus der Gruppe ausgewählt ist, die aus Lithium, Natrium, Kalium, Calcium, Eisen und Mangan besteht.

20. Medizinische Vorrichtung nach Anspruch 1,
wobei das nicht biologisch abbaubare Material ein Nichtmetall aufweist.

21. Medizinische Vorrichtung nach Anspruch 1,
wobei das nicht biologisch abbaubare Material ein Polymer aufweist.

22. Medizinische Vorrichtung nach Anspruch 1,
wobei die medizinische Vorrichtung eine erste Schicht aufweist, die den ersten Polyelektrolyt aufweist, wobei diese erste Schicht vom rohrförmigen Körper getragen wird.

23. Medizinische Vorrichtung nach Anspruch 22,
wobei die erste Schicht eine Dicke von höchstens 500 nm aufweist.

24. Medizinische Vorrichtung nach Anspruch 23,
wobei die erste Schicht eine Dicke von mindestens 0,2 nm aufweist.

25. Medizinische Vorrichtung nach Anspruch 22,
wobei die erste Schicht mindestens eine Öffnung bildet.

26. Medizinische Vorrichtung nach Anspruch 22,
wobei die erste Schicht den rohrförmigen Körper berührt.

27. Medizinische Vorrichtung nach Anspruch 22,
die ferner eine zweite Schicht aufweist, die vom rohrförmigen Körper getragen wird.

28. Medizinische Vorrichtung nach Anspruch 27,
wobei die zweite Schicht die erste Schicht berührt.

29. Medizinische Vorrichtung nach Anspruch 27,
wobei die medizinische Vorrichtung eine Vielzahl von ersten Schichten und eine Vielzahl von zweiten Schichten aufweist.

30. Medizinische Vorrichtung nach Anspruch 29,
wobei mindestens zwei der ersten Schichten durch eine oder mehrere zweite Schichten voneinander beabstandet sind.

31. Medizinische Vorrichtung nach Anspruch 27,
wobei die zweite Schicht einen zweiten Polyelektrolyt aufweist.

32. Medizinische Vorrichtung nach Anspruch 31,
wobei der zweite Polyelektrolyt vom ersten Polyelektrolyt verschieden ist.

33. Medizinische Vorrichtung nach Anspruch 31,
wobei der erste Polyelektrolyt positiv geladen ist und der zweite Polyelektrolyt negativ geladen ist.

34. Medizinische Vorrichtung nach Anspruch 1,
wobei die medizinische Vorrichtung ferner einen zweiten Polyelektrolyt auf dem rohrförmigen Körper aufweist.

35. Medizinische Vorrichtung nach Anspruch 34,
wobei der erste Polyelektrolyt und der zweite Polyelektrolyt miteinander vernetzt sind.

36. Medizinische Vorrichtung nach Anspruch 35,
wobei der erste Polyelektrolyt und der zweite Polyelektrolyt durch mindestens eine kovalente Bindung miteinander vernetzt sind.

37. Medizinische Vorrichtung nach Anspruch 1,
wobei die medizinische Vorrichtung eine Vielzahl von Schichten aufweist.

38. Medizinische Vorrichtung nach Anspruch 37,
wobei ein erster Bereich der medizinischen Vorrichtung eine größere Anzahl von Schichten als ein zweiter Bereich der medizinischen Vorrichtung aufweist.

39. Medizinische Vorrichtung nach Anspruch 38,
wobei der erste Bereich mindestens 20 Schichten aufweist.

40. Medizinische Vorrichtung nach Anspruch 39,
wobei der zweite Bereich mindestens 10 Schichten aufweist.

41. Medizinische Vorrichtung nach Anspruch 37,
wobei die Vielzahl von Schichten eine Gesamtdicke von höchstens 1500 nm hat.

42. Medizinische Vorrichtung nach Anspruch 41,
wobei die Vielzahl von Schichten eine Gesamtdicke von mindestens 5 nm hat.

43. Medizinische Vorrichtung nach Anspruch 37,
wobei die medizinische Vorrichtung mindestens 10 Schichten aufweist.

44. Medizinische Vorrichtung nach Anspruch 43,
wobei die medizinische Vorrichtung höchstens 300 Schichten aufweist.

45. Medizinische Vorrichtung nach Anspruch 1,
wobei die medizinische Vorrichtung eine Endoprothese aufweist.

## Revendications

1. Dispositif médical comprenant :
un corps tubulaire composé d'un matériau biodégradable ; et
un premier polyélectrolyte sur le corps tubulaire.

2. Dispositif médical selon la revendication 1, dans lequel le premier polyélectrolyte est biodégradable.

3. Dispositif médical selon la revendication 1, dans lequel le premier polyélectrolyte comprend un polyacide.

4. Dispositif médical selon la revendication 3, dans lequel le polyacide est choisi dans le groupe comprenant les acides polyphosphoriques, les acides polyvinylsulfuriques, les acides polyvinylsulfoniques, les acides polyvinylphosphoniques, et les acides polyacryliques.

5. Dispositif médical selon la revendication 1, dans lequel le premier polyélectrolyte comprend une polybase.

6. Dispositif médical selon la revendication 5, dans lequel la polybase comprend une polyallylamine, une polyéthylimine, une polyvinylamine, et une polyvinylpyridine.

7. Dispositif médical selon la revendication 1, dans lequel le premier polyélectrolyte comprend un polycation.

8. Dispositif médical selon la revendication 7, dans lequel le polycation est choisi dans le groupe comprenant les polycations poly(allylamine), les polycations polyéthylèneimine, les polycations polydiallyldiméthylammonium, les polycations protamine sulfate, les polycations chitosane, les polycations gélatine, les polycations spermidine, les polycations poly(N-octyl-4-vinyl pyridinium iodure), et les polycations albumine.

9. Dispositif médical selon la revendication 1, dans lequel le premier polyélectrolyte comprend un polyanion.

10. Dispositif médical selon la revendication 9, dans lequel le polyanion est choisi dans le groupe comprenant les polyanions poly(styrène sulfonate), les polyanions acide polyacrylique, les polyanions alginate de sodium, les polyanions eudragit, les polyanions gélatine, les polyanions acide hyaluronique, les polyanions carragheen, les polyanions sulfate de chondroïtine, et les polyanions carboxyméthylcellulose.

11. Dispositif médical selon la revendication 1, dans lequel le premier polyélectrolyte comprend un polymère choisi dans le groupe comprenant l'héparine, les protéines, l'acide polyglycolique, l'acide polylactique, les polyamides, le poly-2-hydroxybutyrate, la polycaprolactone, et l'acide poly(lactique-co-glycolique).

12. Dispositif médical selon la revendication 1, dans lequel le matériau biodégradable comprend un métal.

13. Dispositif médical selon la revendication 1, dans lequel le matériau biodégradable comprend un métal choisi dans le groupe comprenant les métaux alcalins, les métaux alcalinoterreux, le fer, le zinc, et l'aluminium.

14. Dispositif médical selon la revendication 1, dans lequel le matériau biodégradable comprend le fer.

15. Dispositif médical selon la revendication 1, dans lequel le matériau biodégradable comprend le magnésium.

16. Dispositif médical selon la revendication 1, dans lequel le matériau biodégradable comprend un alliage métallique.

17. Dispositif médical selon la revendication 1, dans lequel le matériau biodégradable comprend un alliage de fer.

18. Dispositif médical selon la revendication 1, dans lequel le matériau biodégradable comprend un alliage de magnésium.

19. Dispositif médical selon la revendication 1, dans lequel le matériau biodégradable comprend un premier composant choisi dans le groupe comprenant le magnésium, le titane, le zirconium, le niobium, le tantale, le zinc, et le silicium, et un second composant choisi dans le groupe comprenant le lithium, le sodium, le potassium, le calcium, le fer, et le manganèse.

20. Dispositif médical selon la revendication 1, dans lequel le matériau biodégradable comprend un non-métal.

21. Dispositif médical selon la revendication 1, dans lequel le matériau biodégradable comprend un polymère.

22. Dispositif médical selon la revendication 1, dans lequel le dispositif médical comprend une première couche composée du premier polyélectrolyte, la première couche étant supportée par le corps tubulaire.

23. Dispositif médical selon la revendication 22, dans lequel la première couche a une épaisseur au plus égale à 500 nanomètres.

24. Dispositif médical selon la revendication 23, dans lequel la première couche a une épaisseur au moins égale à 0,2 nanomètre.

25. Dispositif médical selon la revendication 22, dans lequel la première couche définit au moins une ouverture.

26. Dispositif médical selon la revendication 22, dans lequel la première couche vient en contact avec le corps tubulaire.

27. Dispositif médical selon la revendication 22, comprenant en outre une seconde couche qui est supportée par le corps tubulaire.

28. Dispositif médical selon la revendication 27, dans lequel la seconde couche vient en contact avec la première couche.

29. Dispositif médical selon la revendication 27, dans lequel le dispositif médical comprend une pluralité de premières couches et une pluralité de secondes couches.

30. Dispositif médical selon la revendication 29, dans lequel au moins deux des premières couches sont espacées l'une de l'autre par une ou plusieurs secondes couches.

31. Dispositif médical selon la revendication 27, dans lequel la seconde couche comprend un second polyélectrolyte.

32. Dispositif médical selon la revendication 31, dans lequel le second polyélectrolyte est différent du premier polyélectrolyte.

33. Dispositif médical selon la revendication 31, dans lequel le premier polyélectrolyte est chargé positivement et le second polyélectrolyte est chargé négativement.

34. Dispositif médical selon la revendication 1, dans lequel le dispositif médical comprend en outre un second polyélectrolyte sur le corps tubulaire.

35. Dispositif médical selon la revendication 34, dans lequel le premier polyélectrolyte et le second polyélectrolyte forment un réseau réticulé.

36. Dispositif médical selon la revendication 35, dans lequel le premier polyélectrolyte et le second polyélectrolyte forment un réseau réticulé par au moins une liaison covalente.

37. Dispositif médical selon la revendication 1, dans lequel le dispositif médical comprend une pluralité de couches.

38. Dispositif médical selon la revendication 37, dans lequel une première partie du dispositif médical comprend un plus grand nombre de couches qu'une seconde partie du dispositif médical.

39. Dispositif médical selon la revendication 38, dans lequel la première partie comprend au moins 20 couches.

40. Dispositif médical selon la revendication 39, dans lequel la seconde partie comprend au moins 10 couches.

41. Dispositif médical selon la revendication 37, dans lequel la pluralité de couches a une épaisseur totale au plus égale à 1 500 nanomètres.

42. Dispositif médical selon la revendication 41, dans lequel la pluralité de couches a une épaisseur totale au moins égale à 5 nanomètres.

43. Dispositif médical selon la revendication 37, dans lequel le dispositif médical comprend au moins 10 couches.

44. Dispositif médical selon la revendication 43, dans lequel le dispositif médical comprend au plus 300 couches.

45. Dispositif médical selon la revendication 1, dans lequel le dispositif médical comprend une endoprothèse.
